# EUROPEAN PATENT SPECIFICATION

(11) **EP 0 485 219 B1**
(45) Date of publication and mention of the grant of the patent: **13.03.1996**
(21) Application number: 91310322.2
(22) Date of filing: 07.11.1991
(51) Int. Cl.: A61K 31/415, A61K 31/425, A61K 31/445, A61K 31/495, A61K 31/535

(54) **Medicaments for preventing and treating circulatory organ diseases containing spiro-3-heteroazolidine compounds**
Arzneimittel für die Verhütung und Behandlung von Krankheiten der Kreislauforgane enthaltend Spiro-3-heteroazolidin-Verbindungen
Médicaments pour le prévention et le traitement des maladies des organes du système circulatoire contenant des spiro-3-heteroazolidines

(30) Priority: 07.11.1990 JP 301518/90
(43) Date of publication of application: 13.05.1992
(73) Proprietor: SANWA KAGAKU KENKYUSHO CO., LTD., Higashi-ku Nagoya-shi Aichi-ken (JP)
(72) Inventor: Kurono, Masayasu, Sanwa Kagaku, Kenkyusho Co.,Ltd., Nagoya-shi, Aichi-ken (JP); Miura, Kenji, Sanwa Kagaku, Kenkyusho Co.,Ltd., Nagoya-shi, Aichi-ken (JP); Kondo, Yasuaki, Sanwa Kagaku, Kenkyusho Co.,Ltd., Nagoya-shi, Aichi-ken (JP); Usui, Toshinao, Sanwa Kagaku, Kenkyusho Co.,Ltd., Nagoya-shi, Aichi-ken (JP); Hayashi, Motohide, Sanwa Kagaku, Kenkyusho Co.,Ltd, Nagoya-shi, Aichi-ken (JP); Suzuki, Tunemasa, Sanwa Kagaku, Kenkyusho Co.,Ltd, Nagoya-shi, Aichi-ken (JP); Tomiya, Noboru, Sanwa Kagaku, Kenkyusho Co.,Ltd, Nagoya-shi, Aichi-ken (JP); Nakamura, Shigeyoshi, Sanwa Kagaku, Kenkyusho Co., Nagoya-shi, Aichi-ken (JP); Tanaka, Yukiya, Sanwa Kagaku, Kenkyusho Co., Ltd., Nagoya-shi, Aichi-ken (JP); Sawai, Kiichi, Sanwa Kagaku, Kenkyusho Co., Ltd., Nagoya-shi, Aichi-ken (JP)
(74) Representative: Moore, Anthony John

(56) References cited:
- EP-A- 0 193 415
- EP-A- 0 418 834
- INT. CONGR. SER. - EXCERPTA MEDICA, (PROCEEDINGS OF THE JAPAN-US ALDOSE REDUCTASE WORKSHOP, Nagoya, 16th - 17th November 1989), vol. 913, pages 89-96, "Current concepts of aldose reductase and its inhibitions"; K. MIZUNO et al.: "Profile of a new aldose recuctase inhibitor. (2S,4S)-6-fluoro-2',5'-dioxospiro-[chrom an-4,4'-imidazolidine]-2-carboxamide (SNK-860)"
- PATENT ABSTRACTS OF JAPAN, vol. 14, no. 456 (C-765)[4399], 2nd October 1990, page 35 C 765; & JP-A-21 80 846
- PATENT ABSTRACTS OF JAPAN, vol. 10, no. 28 (C326)[2085], 4th February 1986, page 128 C 326; & JP-A-60 181 081
- R. BERKOW et al.: "The Merck Manual of Diagnosis and Therapy", 15th edition, 1987, pages 1380-1381, Merck & Co., Inc., Rahway, NJ, US
- R. BERKOW et al.: "The Merck Manual of Diagnosis and Therapy", 15th edition, 1987, pages 477-501, Merck & Co., Inc., Rahway, NJ, US
- R. BERKOW et al.: "The Merck Manual of Diagnosis and Therapy", 15th edition, 1987, pages 388-389, Merck & Co., Inc., Rahway, NJ, US

## Description

The present invention relates to medicaments for preventing and treating circulatory organ-related diseases (hereinafter called circulatory organ diseases), which contain a spiro-3-heteroazolidine compound as an effective ingredient.

As already known, some spiro-3-heteroazolidine compounds are efficacious for preventing and treating various forms of diabetic complications, typically diabetic cataract, neuropathy and retinopathy, see for instance JP-A-61(1986)-20091, JP-A-63(1988)-57588 and JP-A-63(1988)-126881, and their mode of action is now considered to be due to aldose reductase inhibition.

Many diabetics have been reported to have a critical disposition to circulatory organ diseases - see "A Handbook to the Treatment and Control of Diabetic Blood Vessel Complications", 1st Edition (1987), published by Ishiyaku Shuppan, "Br. Med. J.", Vol. 294, page 1443 (1987) and "N. Engl. Med", Vol. 313, page 1617 (1987). The development of medicaments efficacious against both diabetes and circulatory organ diseases has thus been desirable.

EP-A-0418834, which is prior art within the meaning of Article 54(3) EPC, discloses a composition for treating circulatory diseases which comprises at least one of a certain hydantoin derivative.

We have now found that some novel spiro-3-heteroazolidine compounds are efficacious against not only diabetic complications but also circulatory organ diseases.

The present invention thus provides medicaments for preventing and treating circulatory organ diseases.

More specifically, this invention provides medicament for preventing and treating circulatory organ diseases, which contains as an effective ingredient a spiro-3-heteroazolidine compound represented by the following general formula I; or a pharmacologically acceptable salt or solvate thereof: 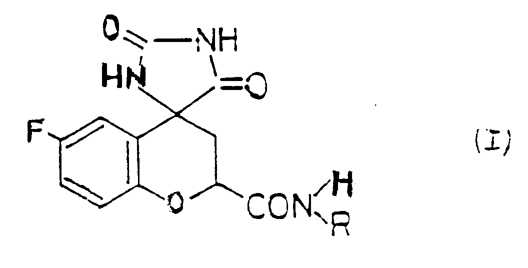
wherein R is selected from 4-methoxyphenyl, -CH₂pyr, or -(CH₂CH₂O)₄CH₃, with pyr representing a pyrrolizidino group, and wherein the compound represented by general formula (I) has (2S, 4S) stereoisomerism.

The spiro-3-heteroazolidine compounds of the Formula I have extremely low toxicity when orally administered, and produce significant activity in inhibiting platelet aggregation, vasodilation, depressing blood pressure, increasing bloodstream, etc. Thus, the compounds of this invention may be efficaciously used for treating, alleviating and preventing various forms of thrombosis, hypertension, cerebrovascular disease and heart disease, typically cerebral infarction, cerebral thrombosis, transient cerebral ischemia, cardiac infarction, coronary thrombosis, arterial sclerosis and stenocardia. For detailed information on the pharmaceutical and pharmacological actions of these compounds, see the experimental Examples to be given later.

The following compounds are more preferably used as the effective ingredient in this invention.
(1) (2S,4S)-6-fluoro-N-[(pyrrolizidin-7a-yl]-2',5'-dioxospiro[chromane-4,4'-imidazolidine]-2-carboxamide
(2) (2S,4S)-6-fluoro-N-(3,6,9,12-tetraoxatridecyl-2',5'-dioxospiro[chromane-4,4'-imidazolidine]-2-carboxamide
For this invention, the compounds represented by Formula I may be available in the form of solvates, e.g. hydrates, or salts.

The salts of the compounds represented by Formula (I) are their pharmaceutically acceptable salts, typically their pharmaceutically acceptable inorganic and organic basic salts such as sodium, lithium, potassium and quaternary ammonium salts as well as diethylamine and diethanolamine salts. These salts may be easily obtained by treating the compounds of Formula I with suitable bases

The compounds represented by Formula (I) have two asymmetric carbon atoms per structure and so can give two stereoisomers and their optical isomers, which may be used alone or in the form of a mixture.

The compounds according to this invention are known as already mentioned, and so may be synthesized by any one of appropriate procedure. For instance, reliance may be placed on procedures disclosed in JP-P-61(1986)-200991, JP-A-63(1988)-57588 and JP-A-1(1989)-242301. Set out below are some preferred reaction schemes: 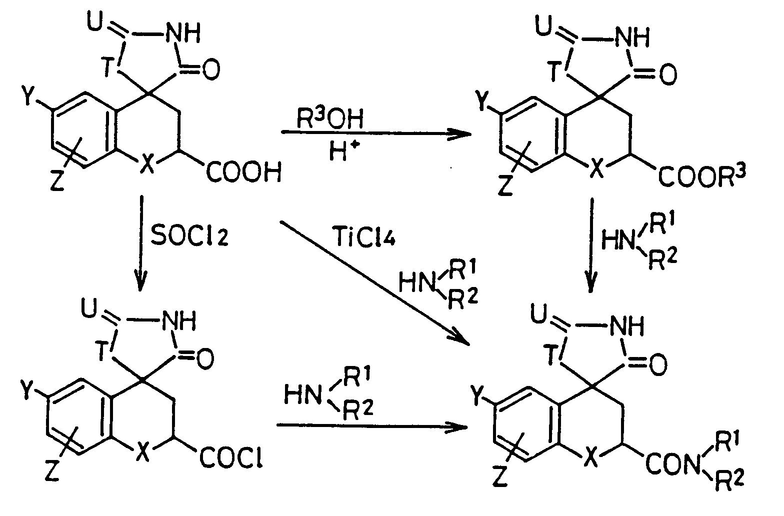
In the formula R³ stands for a lower alkyl group, e.g. an alkyl group having 1 to 4 carbon atoms, T represents a nitrogen atom bonded to a hydrogen atom, Y represents a fluorine atom, Z represents a hydrogen atom and X and U both represent an oxygen atom.

Medicaments for preventing and treating circulatory organ diseases, which contains as an effective ingredient the compound represented by Formula (I) or its pharmacologically acceptable salt or solvate, may be formulated for oral, buccal, topical or per rectal administration. In other words, they may be formulated by conventional procedures in the form of either solid preparations such as tablets, pills, capsules, powders, granules or suppositories or liquid preparations such as solutions, suspensions or emulsions. The solid preparations may be obtained using such vehicles or excipients as starch, lactose, glucose, calcium phosphate, magnesium stearate or gum arabic, optionally with lubricants, binders, disintegrators, colorants or the like. The liquid preparations may be obtained using stabilizers, dissolution aids, suspending agents, emulsifiers, buffers, preservatives and the like.

The compound of this invention or its salt or solvate may be administered to patients with circulatory organ diseases by either oral or parenteral (e.g. intramuscular, hypodermic and intravenous) routes. The dose may be determined case-by-case depending upon what type and form of preparation are used, what conditions patients are in, how old they are and other significant factors. Usually, note that human adults may receive a dose of about 0.1 to 2000 mg, particularly about 1 to 500 mg a day and one to three times on daily basis.

### EXAMPLES

The present invention will now be illustrated by the following Examples.

### Preparation of Compounds

Procedures similar to those set forth in the examples of the aforesaid specifications were followed to prepare Compounds 1a to 9a wherein X = O, Y = F, Z = H, T = hydrogen-substituted nitrogen (HN) and U = O, as set out in Table 1.

**Table 1**

| Compounds | Stereoisomerism | CONR¹R² | M.P. in |
|---|---|---|---|
| 1a | (2RS,4RS) | CONHBu | 286-288 |
| 1b | (2S,4S) | CONHBu | 265-266 |
| 2a | (2RS,4RS) | CONHPh(4-OMe) | 301-304 |
| 2b | (2S,4S) | CONHPh(4-OMe) | 190-191 |
| 3a | (2RS,4RS) | CONHCH₂pyr | 210-212 |
| 3b | (2S,4S) | CONHCH₂pyr | 137-150 |
| 4a | (2RS,4RS) | CONH(CH₂CH₂O)₄CH₃ | 162-164 |
| 4b | (2S,4S) | CONH(CH₂CH₂O)₄CH₃ | 135-137 |
| 5a | (2RS,4RS) | CONH₂ | 286-300 |
| 5b | (2S,4S) | CONH₂ | 290-291 |
| 5c | (2R,4R) | CONH₂ | 242-243 |
| 5d | (2RS,4RS) | CONH₂ | 285-295 |
| 6a | (2RS,4RS) | CONHMe | 297-300 |
| 7a | (2RS,4RS) | CONHMe₂ | 285-293 |
| 8a | (2RS,4RS) | CONHEt | 300 or more |
| 9a | (2RS,4RS) | CONHPr | 282-284 |
| pyr: pyrrolizidino group | | | |

### Test Example 1 : In Vitro Action on Inhibiting Platelet

### Aggregation

Added to blood gathered from the descending aorta of a lightly etherized rat was citric acid (3.8 % sodium citrate) in a volume ratio of 1/10, and the blood was centrifuged at 4 and 1500 rpm for 10 minutes to obtain a supernatant fluid in the form of platelet rich plasma (PRP for short). Then, the residues were centrifuged to obtain a supernatant fluid in the form of platelet poor plasma (PPP for short). For measurement, an amount of PRP collected from two to four animals was pooled. The platelet aggregation test was carried out with NBS HEMA TRACER (Model PAT-60 made by Niko Bioscience Co., Ltd.). 267 µml of PRP were put in a PRP cuvette having a stirrer bar with the transmittance set at 0%, while 300 µl of PPP were put in a PPP cuvette with the transmittance set at 100%. After 1-minute preincubation, 3 µl of the test compound dissolved in dimethyl sulfoxide were added and, one minute later, 30 µl of an aggregation inducer (collagen) were introduced. Added to a control group was the test compound together with 3 µl of dimethyl sulfoxide. Upon the addition of the aggregation inducer, a maximum transmittance change was read to find a platelet aggregation change in %. An inhibition in % relative to the control - 100% was found to give the 50% inhibition concentration (IC₅₀). Table 2 sets out the results, which indicate that the present compounds have a significant action on inhibiting platelet aggregation.

**Table 2**

| Compounds | | IC₅₀ (M) |
|---|---|---|
| Indomethacin | | 6.8 x 10⁻⁵ |
| Aspirin | | 2.1 x 10⁻⁴ |
| Compound | 1b | 1.3 x 10⁻⁴ |
| | 2b | 4.3 x 10⁻⁴ |

### Test Example 2 : Ex Vivo Actions on Inhibiting

Platelet Aggregation and Poikilocytes

### 1) Administration and Blood Gathering

Five minutes after the test compound had been intravenously administered to the tail of a rat, blood was gathered from the descending aorta of the rat which was lightly etherized. Added to this blood was citric acid (3.8% sodium citrate) in a volume ratio of 1/10.

### 2) Determination of Platelet Aggregation

The thus treated blood was centrifuged at 1500 rpm for 5 minutes to obtain a supernatant fluid in the form of PRP. The residues were then again centrifuged at 4°C and 3000 rpm for 10 minutes to obtain a supernatant fluid in the form of PPP. For the platelet aggregation test, NAB HEMA TRACER 601 (Model PAT-60 made by Niko Bioscience Co., Ltd.) was used. 180 µml of PRP were put in a PRP cuvette having a stirrer bar with the transmittance set at 0%, while 300 µl of PPP were put in a PPP cuvette with the transmittance set at 100%. After 1-minute preincubation, 20 µl of an aggregation inducer (collagen or ADP) were introduced.

A control group, to which dimethylacetamide and tartaric acid in a ratio of 2/3 were given in a quantity of 1 ml/kg, was treated similarly to the test group. Upon the addition of the aggregation inducer, a maximum transmittance change was read to find a platelet aggregation change in %. An inhibition in % relative to the control - 100% was found to calculate the 50% inhibition concentration (IC₅₀). Table 3 sets out the results, from it is noted that the present compounds have a significant action on inhibiting platelet aggregation.

**Table 3**

| Ex Vivo Action on Platelet Aggregation | | | |
|---|---|---|---|
| Compounds | Conc. (mg/kg) | ADP (1 x 10⁻⁶M) | Collagen (10 M) |
| Compound 1b | 3.0 | 44 | 73* |
| Aspirin | 30.0 | - | 77 |
| Indomethacin | 30.0 | - | 49 |

| | | | |
|---|---|---|---|
| *;p<0.05 | | | |

### 3) Determination of Poikilocytes

Blood gathered with the addition of citric acid was centrifuged at 4°C and 1500 rpm for 10 minutes. The supernatant fluid was discarded. Added to the residues was physiological saline in an amount of about 1.5 times as great, again followed by 10-minute centrifugation at 4°C and 1500 rpm. The supernatant fluid was discarded. One hundred (100) µl of erythrocytes left upon such procedures repeated twice were diluted with physiological saline to prepare 50 ml of a 0.2 % erythrocyte suspension. This suspension was incubated at 37°C for 5 minutes, cooled down to room temperature and examined to find how much the poikilocytes were ameliorated by the filtration-under-pressure procedures described by F. Katsuraya in "Geriatric Medicine", Vol. 20, pp. 151-156 (1982).

To this end, the suspension sample was passed from a continuous feeder through a Millipore membrane having a mean pore size of 5 µl at a rate of 5 ml/min, during which the pressure on the upstream side of the membrane was measured through a pressure transducer with a pressure amplifier and, at the same time, was recorded on a polygraph chart sheet.

The results, set out in Table 4, indicate that the present compound has a significant action upon ameliorating poikilocytes.

**Table 4**

| Ex Vivo Action on Ameliorating Poikilocytes | | |
|---|---|---|
| Compound | Conc. in mg/kg | Amelioration in % |
| Compound 1b | 0.1 | 24.1 |

### Test Example 3 - Action on Extracted Guinea Pigs' Aortae

Stunned by receiving a blow on the head Hartley guinea pigs (weighing 300-500 g) were fixed at the back regions, and the thoracic aortae were then extracted to prepare helical samples, each ca. 2 mm in width and ca. 25 mm in length. Each sample was suspended in a Magnus tube under a load of ca. 1 g and connected at the upper end with an FD pickup by means of silk thread to record its isometric tension change on a recorder. Note that the Magnus tube contained 10 ml of a Krebs-Henseleit solution and was held at 37°C with the supply of 95% O₂-5% CO₂ gases, said Krebs-Henseleit solution consisting of, in mM, the following ingredients:
NaCl: 118, KCl: 4.7, CaCl₂: 2.55, MgSO₄: 1.18, KH₂PO₄: 1.18, NaHCO₃: 24.88 and glucose: 11.1
Prior to initiating the experiment, the sample was allowed to stand for 60 to 90 minutes, during which the Krebs-Henseleit solution was replaced by a new one at an interval of 20 to 30 minutes. After the tension recorded had been found to be kept stable, KCl was added to obtain a final concentration of 2.5 x 10⁻²M or norepinephrine (NE for short) was added to obtain a final concentration of 10⁻⁶ g/ml. After the contraction of the sample had been kept constant, the test compound was added at an incremental rate from the concentration of ca. 10⁻⁸ to 10⁻⁶M to observe what reaction took place. Finally, papaverine was additionally provided to regulate the final concentration to 2 x 10⁻⁵g/ml, thereby observing and recording what relaxing reaction took place.

The state stabilized by the addition of KCl or NE was defined as a 100% relaxation rate, on the basis of which what reaction took place at each concentration was relatively estimated. For the purpose of data analysis, the 50% relaxation rates (IC₅₀) were found.

The results are reported in Table 5.

**Table 5**

| Compounds | | IC₅₀(M) | |
|---|---|---|---|
| | | Contraction by KCl | Contraction by NE |
| Compound | 1b | 8.0 x 10⁻⁶ | > 10⁻³ |
| | 2b | > 10⁻³ | > 10⁻³ |
| | 3b | 2.0 x 10⁻⁵ | 2 x 10⁻⁴ |
| | 4b | 2.7 x 10⁻⁴ | 2 x 10⁻⁴ |
| Cinepazid | | 2.0 x 10⁻³ | 1 x 10⁻³ |
| Cinnarizine | | 2.0 x 10⁻⁴ | 1 x 10⁻⁴ |

### Test Example 4 - Action on Extracted Guinea Pigs' Hearts

Stunned by receiving a blow on the heads, Hartley guinea pigs (weighing 300-400 g) were fixed on the back regions to extract their hearts. Each of the extracted hearts was placed in a beaker containing the same Krebs-Henseleit solution as used in Test Example 4 and supplied with 95% O₂-5% CO₂ gases, thereby stripping it of blood deposits, and was then passed into a sample forming glass vessel. This vessel was previously filled with a Krebs-Henseleit solution and the above gaseous mixture continued to be supplied thereto during preparing the sample.

In order to prepare the sample, the right and left atria were cut out of the ventricular muscle along the atriventricular sulcus from the center of both the right and left auricles.

### (a) Right Atrium Sample

The thus extracted right atrium was suspended in a Magnus tube with a tension of 0.2 to 0.3 g, said tube supplied with 95% O₂ - 5% CO₂ gases, containing 10 ml of a Krebs-Henseleit solution and maintained at 32±1°C. The contraction of the sample was recorded on a recorder through an FD pickup simultaneously with counting and recording the beat with an instantaneous cardiograph unit.

### (b) Left Atrium Sample

The extracted left atrium was fixed with a micro wire clamp through both the auricles in a Magnus tube supplied with 95% O₂ - 5% CO₂ gases, containing 30 ml of a Krebs-Henseleit solution and maintained at a solution temperature of 32±1°C, and then suspended therein under a checking tension of ca. 0.25 g. While a platinum bipolar electrode attached to the above-mentioned micro wire clamp was in contact with the atrium, stimuli were given to it by 1-Hz, 2-msec. and 10-V rectangular waves derived through an electrical stimulator and an isolator to record the movement of said atrium with an FD pickup connected with it through silk thread.

The actions of the present and control compounds on the right (counting the heart rate) and left (measuring the systole force) atria are reported below.

**Table 6**

| Compounds | | Heart Rates | | Systole Forces | |
|---|---|---|---|---|---|
| | | 10⁻⁵(M) | 10⁻⁴(M) | 10⁻⁵(M) | 10⁻⁴(M) |
| Compound 1b | | - | - | - | + |
| Control | A | ++ | ++ | ++ | ++ |
| | B | ++ | ++ | - | + |
| Note that A and B stands for propranolol and ifenprodil, respectively, and that ++ indicates 20% or more suppression, + 10-20% suppression and - 0% suppression. | | | | | |

### Pharmaceutical Example 1

The ingredients, shown in Table 7, were blended together and tableted conventionally to prepare 1000 tablets for oral administration. These tablets each contain 50 mg of the effective ingredient.

**Table 7**

| Ingredients | Amounts in g |
|---|---|
| Effective Ingredient (Compound 1b) | 50 |
| Sodium citrate | 25 |
| Arginine | 10 |
| Polyvinylpyrrolidone | 10 |
| Magnesium stearate | 5 |

## Claims (Claims for the following Contracting State(s): AT, BE, CH, LI, DE, DK, FR, GB, GR, IT, LU, NL, SE)

1. A medicament for use in preventing and treating diseases of the circulatory organs, which contains as an effective ingredient a spiro-3-heteroazolidine compound represented by the following general formula (I); pharmacologically acceptable salt or solvate thereof: 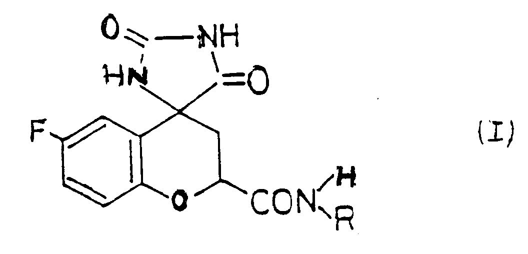 wherein R is selected from 4-methoxyphenyl, -CH₂pyr, or -(CH₂CH₂O)₄CH₃, with pyr representing a pyrrolizidino group, and wherein the compound represented by general formula (I) has (2S, 4S) stereoisomerism.

2. A medicament as claimed in Claim 1, wherein the circulatory organ diseases are thrombosis, hypertension, cerebrovascular disease or a heart disease.

3. A medicament as claimed in Claim 1 or 2, wherein the effective ingredient is either:
a) (2S,4S)-6-fluoro-N-[(pyrrolizidin-7a-yl]-2',5'-dioxospiro[chromane-4,4'-imidazolidine]-2-carboxamide; or
b) (2S,4S)-6-fluoro-N-(3,6,9,12-tetraoxatridecyl-2',5'-dioxospiro[chromane-4-4'-imidazolidine]-2-carboxamide; or their pharmacologically acceptable salts.

4. The use, in the manufacture of a medicament for treating diseases of the circulatory organs, of a spiro-3-heteroazolidine having the general formula (I) as set forth and defined in Claim 1.

## Claims (Claims for the following Contracting State(s): ES)

1. A process for preparing a medicament for use in preventing and treating diseases of the circulatory organs, which comprises mixing as an effective ingredient a spiro-3-heteroazolidine compound represented by the following general formula (I); pharmacologically acceptable salt or solvate thereof: 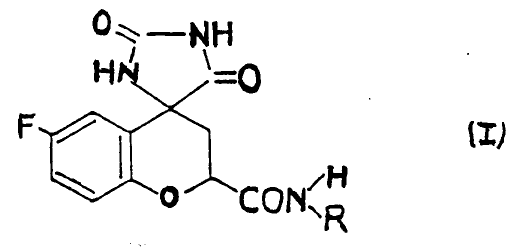 wherein R is selected from 4-methoxyphenyl, -CH₂pyr, or -(CH₂CH₂O)₄CH₃, with pyr representing a pyrrolizidino group, and wherein the compound represented by general formula (I) has (2S, 4S) stereoisomerism, with at least one pharmacologically acceptable carrier or excipient.

2. A process as claimed in Claim 1, wherein the circulatory organ diseases are thrombosis, hypertension, cerebrovascular disease or a heart disease.

3. A process as claimed in Claim 1 or 2, wherein the effective ingredient is either:
a) (2S,4S)-6-fluoro-N-[(pyrrolizidin-7a-yl]-2',5'-dioxospiro[chromane-4,4'-imidazolidine]-2-carboxamide; or
b) (2S,4S)-6-fluoro-N-(3,6,9,12-tetraoxatridecyl-2',5'-dioxospiro[chromane-4-4'-imidazolidine]-2-carboxamide; or their pharmacologically acceptable salts.

4. The use, in the manufacture of a medicament for treating diseases of the circulatory organs, of a spiro-3-heteroazolidine having the general formula (I) as set forth and defined in Claim 1.

## Patentansprüche (Patentansprüche für folgende(n) Vertragsstaat(en): AT, BE, CH, LI, DE, DK, FR, GB, GR, IT, LU, NL, SE)

1. Arzneimittel zur Verhütung und Behandlung von Krankheiten der Kreislauforgane, enthaltend als Wirkstoff eine Spiro-3-heteroazolidin-Verbindung entsprechend der nachstehenden allgemeinen Formel (I), eines pharmakologisch zulässigen Salzes oder Solvats davon: 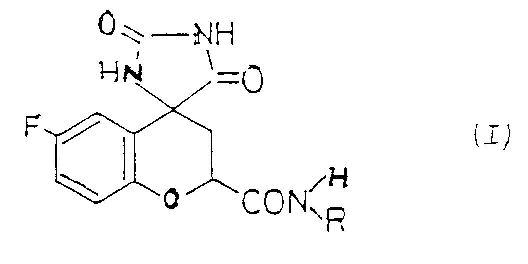 wobei R ausgewählt ist aus 4-Methoxyphenyl, -CH₂pyr, oder -(CH₂CH₂O)₄CH₃, wobei pyr eine Pyrrolizidinogruppe repräsentiert und wobei die durch die allgemeine Formel (I) wiedergegebene Verbindung (2S, 4S) Stereoisomerie zeigt.

2. Arzneimittel nach Anspruch 1, wobei die Kreislauforganerkrankungen Thrombose, Hypertension, cerebrovasculare Krankheit oder eine Herzkrankheit sind.

3. Arzneimittel nach Anspruch 1 oder 2, bei der der Wirkstoff entweder:
a) (2S,4S)-6-fluor-N-[(pyrrolizidin-7a-yl]-2',5'-dioxospiro[chroman-4,4'-imidazolidin]-2-carboxamid; oder
b) (2S,4S)-6-fluoro-N-(3,6,9,12-tetraoxatridecyl-2',5'-dioxospiro[chroman-4-4'-imidazolidin]-2-carboxamid; oder eines ihrer pharmakologisch zulässigen Salze ist.

4. Verwendung eines Spiro-3-heteroazolidins mit der allgemeinen Formel (I), wie sie im Anspruch 1 definiert worden ist, bei der Herstellung eines Arzneimittels zur Behandlung von Krankheiten der Kreislauforgane.

## Patentansprüche (Patentansprüche für folgende(n) Vertragsstaat(en): ES)

1. Ein Verfahren zur Herstellung eines Medikaments zum Gebrauch bei der Verhütung und Behandlung von Krankheiten der am Blutkreislauf beteiligten Organe, das sich auf das Mischen als Wirkstoff einer durch die nachstehende allgemeine Formel (I) 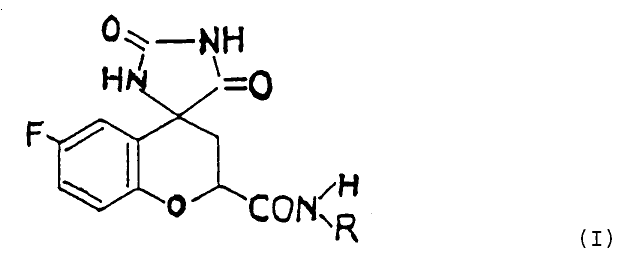 dargestellten Spiro-3-heteroazolidinverbindung bzw. eines pharmakologisch annehmbaren Salzes oder Solvats davon erstreckt, wobei R unter 4-Methoxyphenyl, -CH₂pyr oder -(CH₂CH₂O)₄CH₃ ausgewählt ist, während pyr eine Pyrrolizidingruppe bezeichnet, und wobei die durch die allgemeine Formel (I) dargestellte Verbindung (2S, 4S)-Stereoisomerie aufweist, während mindestens ein pharmakologisch annehmbarer Trägerstoff oder Arzneimittelträger vorhanden ist.

2. Ein Verfahren nach Anspruch 1, bei dem die Krankheiten der am Blutkreislauf beteiligten Organe Thrombose, erhöhter Blutdruck, Krankheit der Hirngefäße oder eine Herzkrankheit sind.

3. Ein Verfahren nach Anspruch 1 oder 2, bei dem der Wirkstoff entweder
a) (2S,4S)-6-Fluoro-N-[(pyrrolizidin-7a-yl]-2',5'-dioxospiro[chroman-4,4'-imidazolidin]-2-carboxamid; oder
b) (2S,4S)-6-Fluoro-N-(3,6,9,12-tetraoxatridecyl-2',5'- dioxospiro[chroman-4-4'-imidazolidin]-2-carboxamid; oder eines von deren pharmakologisch annehmbaren Salzen ist.

4. Der Gebrauch bei der Herstellung eines Medikaments zur Behandlung von Krankheiten der am Blutkreislauf beteiligten Organe eines Spiro-3-heteroazolidins mit der allgemeinen Formel (I), wie diese in Anspruch 1 angeführt und definiert ist.

## Revendications (Revendications pour l'(les) Etat(s) contractant(s) suivant(s): AT, BE, CH, LI, DE, DK, FR, GB, GR, IT, LU, NL, SE)

1. Un médicament à utiliser dans la prévention et le traitement des maladies des organes circulatoires, lequel contient comme ingrédient actif une spiro-3-heteroazolidine représentée par la formule générale (I) suivante ; un sel ou un solvate pharmacologiquement acceptable de celle-ci : 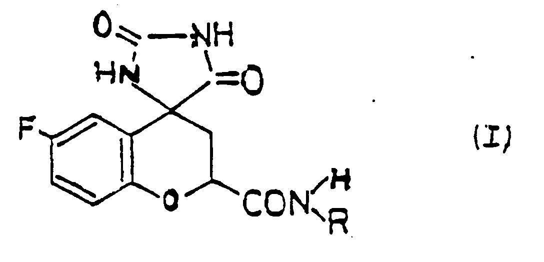 dans laquelle R est sélectionné parmi le 4-méthoxyphényle, le -CH₂pyr, ou le -(CH₂CH₂O)₄CH₃, pyr représentant un groupe pyrrolizidine, et dans lequel le composé représenté par la formule générale (I) a une stéréoisométrie (2S, 4S).

2. Un médicament selon la Revendication 1, dans lequel les maladies des organes circulatoires sont la thrombose, l'hypertension, une maladie cérébro-vasculaire ou une maladie de coeur.

3. Un médicament selon la Revendication 1 ou 2, dans lequel l'ingrédient actif est :
a) le (2S,4S)-6-fluoro-N-[(pyrrolizidin-7a-yl]-2',5'-dioxospiro[chromane-4,4'-imidazolidine]-2-carboxamide ; ou
b) le (2S,4S)-6-fluoro-N-(3,6,9,12-tétraoxatridécyl-2',5'-dioxospiro[chromane-4,4'-imidazolidine]-2-carboxamide ;
ou leurs sels pharmacologiquement acceptables.

4. L'utilisation, dans la fabrication d'un médicament pour le traitement des maladies des organes circulatoires, d'une spiro-3-heteroazolidine ayant la formule générale (I) telle que présentée et définie dans la Revendication 1.

## Revendications (Revendications pour l'(les) Etat(s) contractant(s) suivant(s): ES)

1. Procédé de préparation d'un médicament à utiliser dans la prévention et le traitement des maladies des organes circulatoires, qui comprend comme ingrédient actif un composé de spiro-3-hétéroazolidine représenté par la formule générale suivante (I) ; un sel ou un solvate pharmacologiquement acceptable de celle-ci : 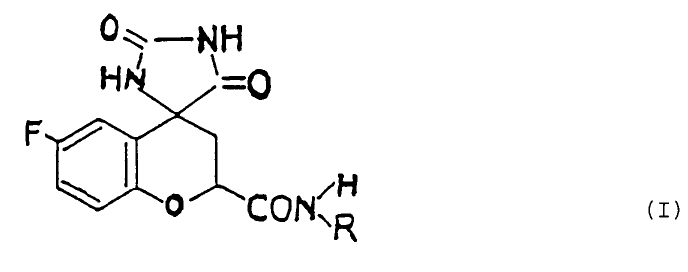 où R est sélectionné parmi le 4-méthoxyphényle, le -CH₂pyr, ou le -(CH₂CH₂O)₄CH₃, pyr représentant un groupe pyrrolizidine, et où le composé représenté par la formule générale (I) a une stéréoisométrie (2S, 4S), avec au moins un véhicule ou excipient pharmacologiquement acceptable.

2. Procédé selon la Revendication 1, où les maladies des organes circulatoires sont la thrombose, l'hypertension, une maladie cérébro-vasculaire ou une maladie de coeur.

3. Procédé selon la Revendication 1 ou 2, dans lequel l'ingrédient actif est soit :
a) le (2S,4S)-6-fluoro-N-[(pyrrolizidin-7a-yl]-2',5'-dioxospiro[chromane-4,4'-imidazolidine]-2-carboxamide ; soit
b) le (2S,4S)-6-fluoro-N-(3,6,9,12-tétraoxatridécyl-2',5'-dioxospiro[chromane-4-4'imidazolidine]-2-carboxamide ; soit leurs sels pharmacologiquement acceptables.

4. Utilisation, dans la fabrication d'un médicament pour le traitement des maladies des organes circulatoires, d'une spiro-3-hétéroazolidine ayant la formule générale (I) présentée et définie dans la Revendication 1.
